# DEMANDE DE BREVET EUROPEEN

(11) **EP 4 272 770 A1**
(43) Date de publication de la demande: **08.11.2023**
(21) Numéro de dépôt: 23170476.8
(22) Date de dépôt: 27.04.2023
(51) Int. Cl.: A61L 9/20, B60H 1/00, F04D 25/08, F04D 29/60, F04D 29/70, F24F 8/22

(54) **BLOC DE TRAITEMENT D'AIR POUR ÉQUIPEMENT DE VENTILATION**

(30) Priorité: 04.05.2022 FR 2204236
(71) Demandeur: ALSTOM Transport SA, 93400 St-Ouen sur Seine (FR)
(72) Inventeur: BEBON, Dominique, 67720 WEYERSHEIM (FR); RANDRIANARISOA, Hélian, 67210 GOXWILLER (FR); XOLIN, Alexandre, 67120 MOLSHEIM (FR)
(74) Mandataire: Cabinet Nuss

(57) **Abrégé**

L'invention concerne un bloc de traitement d'air (1) réalisé sous la forme d'une cartouche (2) configurée pour être positionnée sur au moins une partie du parcours de circulation d'air dans un équipement de ventilation, ce bloc de traitement (1) comprenant un volume intérieur (21) associé à au moins un orifice d'entrée (3) et au moins un orifice de sortie (4) positionnés aux extrémités d'un parcours de circulation d'air, caractérisé en ce que au moins une partie de la surface intérieure du volume (21) est équipée d'au moins une source d'émission (5) de lumière de longueur d'onde comprise entre 100 nm et 400 nm arrangée pour rayonner vers l'intérieur du volume du bloc (1) au niveau d'au moins une section du parcours de circulation d'air à l'intérieur du volume du bloc (1).

## Description

La présente invention se rapporte au domaine des dispositifs de ventilation pour véhicule et plus particulièrement au domaine des dispositifs de ventilation permettant un traitement de l'air ventilé.

Les véhicules de transport en commun forment des structures sensiblement confinées à l'intérieur desquelles les usages sont amenés à se rencontrer, dans le cadre notamment de densités ponctuelles de population susceptibles d'être particulièrement importante, en particulier lors de périodes de fortes affluences. Cette affluence et le flux de passagers à l'intérieur du véhicule, dans une certaine promiscuité, réalisent un terrain favorable à la circulation et à la transmission de virus et de germes.

Bien que les portes du véhicule soient régulièrement ouvertes au niveau de points d'arrêt pour le changement de passagers, ces ouvertures de portes sont réalisées pendant des périodes restreintes qui ne permettent pas d'opérer l'introduction d'un volume important d'air frais et donc un renouvellement suffisant de l'air de l'habitacle du véhicule. Aussi, pour améliorer ce renouvellement, des mécanismes de ventilation sont intégrés au véhicule de façon à permettre, d'une part, un brassage de l'air intérieur du véhicule et, d'autre part, une importation d'air à l'intérieur de l'habitacle depuis l'extérieur du véhicule. Toutefois, la ventilation du véhicule n'est généralement pas suffisante pour que le volume d'air introduit dans l'habitacle soit suffisant pour un renouvellement rapide de l'air intérieur de l'habitacle. En conséquence, en présence de virus et/ou de germes capables de persistance en milieu fermé et à des températures tempérées, de tels mécanismes de ventilation ne sont pas en mesure de réduire, sinon d'empêcher une contamination des passagers par voies respiratoires.

La présente invention a pour but de pallier ces inconvénients en proposant une solution permettant de limiter, voire d'éviter, les risques de contamination de passagers d'un véhicule par un germe et/ou un virus susceptible d'être en suspension dans l'atmosphère, tout en étant adaptable et/ou intégrable dans des mécanismes de ventilation existants de véhicules.

L'invention se rapporte à un bloc de traitement d'air réalisé sous la forme d'une cartouche configurée pour être positionnée sur au moins une partie du parcours de circulation d'air dans un équipement de ventilation, ce bloc de traitement comprenant un volume intérieur associé à au moins un orifice d'entrée et au moins un orifice de sortie positionnés aux extrémités d'un parcours de circulation d'air, caractérisé en ce que au moins une partie de la surface intérieure du volume est équipée d'au moins une source d'émission de lumière de longueur d'onde comprise entre 100 nm et 400 nm arrangée pour rayonner vers l'intérieur du volume du bloc au niveau d'au moins une section du parcours de circulation d'air à l'intérieur du volume du bloc.

L'invention concerne également un dispositif de ventilation qui intègre au moins un bloc de traitement selon l'invention.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :

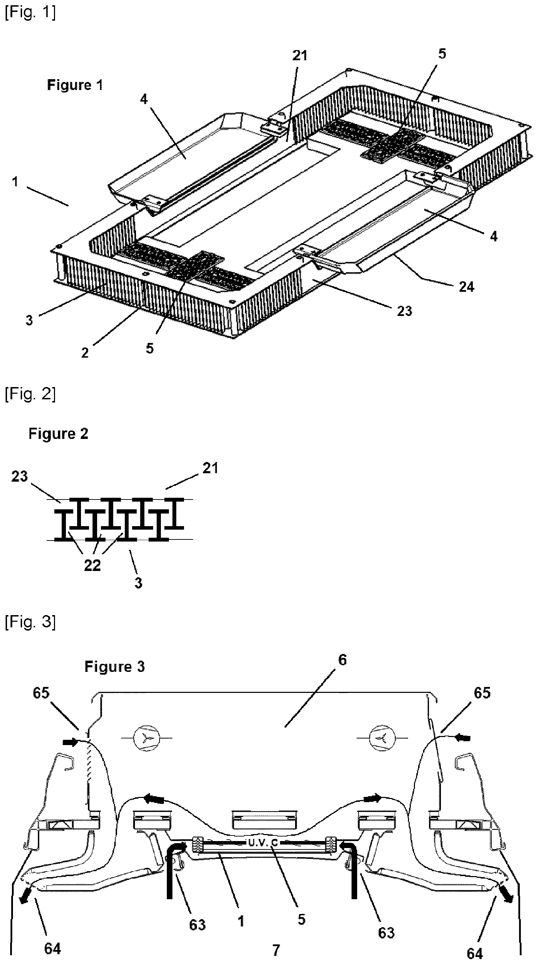

[Fig. 1] représente une illustration schématique d'un exemple de bloc de traitement d'air selon l'invention,

[Fig. 2] représente une illustration schématique d'un exemple d'arrangement en chicanes au niveau d'un cadre périphérique d'un exemple de bloc de traitement d'air selon l'invention,

[Fig. 3] représente une illustration schématique d'un exemple de dispositif de ventilation intégrant un bloc de traitement d'air selon l'invention.

L'invention se rapporte à un bloc de traitement d'air 1 réalisé sous la forme d'une cartouche 2 configurée pour être positionnée sur au moins une partie du parcours de circulation d'air dans un équipement de ventilation, ce bloc de traitement 1 comprenant un volume intérieur 21 associé à au moins un orifice d'entrée 3 et au moins un orifice de sortie 4 positionnés aux extrémités d'un parcours de circulation d'air, caractérisé en ce que au moins une partie de la surface intérieure du volume 21 est équipée d'au moins une source d'émission 5 de lumière de longueur d'onde comprise entre 100 nm et 400 nm arrangée pour rayonner vers l'intérieur du volume du bloc 1 au niveau d'au moins une section du parcours de circulation d'air à l'intérieur du volume du bloc 1.

Le bloc de traitement d'air 1 de l'invention réalise ainsi un conduit de traitement au travers duquel un volume d'air est mis en circulation, de sorte que ce volume d'air soit amené à être traversé par au moins une partie du rayonnement de la source d'émission 5 de lumière. Cette source d'émission 5 de lumière est spécifiquement sélectionnée pour diffuser un rayonnement de longueur d'onde comprise entre 100 nm et 400 nm, c'est-à-dire un rayonnement de type ultraviolet, disposant de propriétés germicide et virucide. De façon préférée, les rayonnement ultra-violet fait intervenir des UV de type C et/ou un rayonnement de longueur d'onde comprise entre 250 et 270 nm. Le rayonnement de type ultraviolet est en mesure d'être absorbé par les acides nucléiques, ARN et ADN, qui composent le matériel génétique des germes et virus, de sorte que ce matériel génétique est en mesure d'être dégradé. Aussi, les germes et virus en suspension dans l'air qui circule dans le bloc de traitement 1 sont en mesure d'avoir leur matériel génétique dégradé par le rayonnement ultraviolet et d'être ainsi détruits. Le flux d'air au niveau d'une sortie 4 du bloc de traitement 1 se trouve ainsi en mesure d'être dépourvu de germes et virus. Selon la longueur de la course du flux d'air au travers du rayonnement ultraviolet et selon l'intensité de ce rayonnement, la dégradation du matériel génétique des germes et virus en suspension sera en mesure d'être plus accentuée.

Il convient de relever que le bloc de traitement 1 selon l'invention est dépourvu de mécanisme de ventilation spécifiquement dédié. Ce bloc de traitement 1 ayant vocation à être intégré dans un équipement de ventilation, il est configuré pour profiter du flux d'air généré par l'équipement de ventilation pour agir sur les germes et virus en suspension dans l'air en circulation sans qu'aucun mécanisme de ventilation propre au bloc de traitement 1 ne soit nécessaire. Cette absence de mécanisme de ventilation supplémentaire intégré au bloc de traitement 1 permet également de limiter le volume sonore de fonctionnement lier à la motorisation de la circulation de l'air.

Selon un exemple de construction se rapportant à une variante de l'invention, le bloc de traitement d'air 1 est caractérisé en ce que au moins une partie de la surface intérieure du volume intérieur 21 est réalisée sous la forme d'une surface adaptée à la réflexion d'au moins une partie du rayonnement de longueur d'onde comprise entre 100 nm et 400 nm, de sorte que ce rayonnement converge au niveau d'au moins une section du parcours de circulation d'air à l'intérieur du volume 21 du bloc 1. Le positionnement de surfaces réfléchissantes dans le volume intérieur 21 du bloc de traitement d'air 1 permet d'optimiser la distribution du rayonnement à l'intérieur du volume 21 du bloc 1 à partir d'une source unique de rayonnement. En effet, la réflexion du rayonnement à l'intérieur du volume intérieur permet de limiter les pertes d'énergies en concentrant le rayonnement sur le circuit du flux d'air à l'intérieur du bloc de traitement 1. Grâce aux surfaces réfléchissantes, un même rayon est ainsi en mesure de traverser plusieurs fois le flux d'air dans le bloc de traitement 1, de sorte que les chances d'impact d'un germe ou d'un virus en suspension dans l'air et donc d'effet du rayonnement sur son matériel génétique se trouvent multipliées. Dans le volume intérieur 21 du bloc 1, l'efficacité du rayonnement apporté par une source d'émission 5 unique de lumière pour le traitement du flux d'air en circulation se trouve ainsi optimisée. Les différentes surfaces réfléchissantes du volume intérieur 21 du bloc de traitement d'air 1 sont susceptibles de correspondre à des surface planes ou, alternativement, des surfaces portant une convexité ou une concavité. De façon complémentaire, ces surfaces sont susceptibles d'être réalisées par une combinaison de portions de surface planes et/ou convexes et/ou concaves. Selon un exemple de construction, ces surfaces de réflexion sont réalisées sous la forme d'une peinture ou d'un revêtement par exemple de type miroir ou chromé ou encore type aluminium.

Selon un exemple de construction se rapportant à une autre variante de réalisation de l'invention susceptible d'être combinée avec les différentes variantes de réalisation précédentes, le bloc de traitement d'air 1 est caractérisé en ce que le parcours de circulation d'air à l'intérieur du volume du bloc 1 comprend au moins une chicane 22 positionnée dans le volume intérieur 21, de sorte que aucun rayon provenant de la au moins une source d'émission 5 de lumière soit en mesure de traverser le au moins un orifice d'entrée 3 et/ou le au moins un orifice de sortie 4 du bloc de traitement 1. La chicane 22 est disposée à l'intérieur du bloc de traitement 1 de sorte que l'une de ses parois soit en mesure d'empêcher une émission de lumière de longueur d'onde comprise entre 100 nm et 400 nm vers l'extérieur du bloc de traitement d'air 1. Aussi, l'effet du rayonnement UV des sources d'émission 5 du bloc de traitement 1 sur des acides nucléiques se trouve strictement circonscrit au volume intérieur 21. Seuls les éléments présents dans le volume intérieur 21 du bloc de traitement 1, notamment en suspension dans le flux d'air en circulation, sont soumis au rayonnement UV. Le risque d'effet de ce rayonnement UV à l'extérieur du bloc de traitement se trouve écarté. Ainsi, l'intégration d'un bloc de traitement 1 selon l'invention dans un véhicule, notamment dans un environnement accessible à des passagers ou usagers, est en mesure de fonctionner sans qu'aucun rayonnement UV émis à l'intérieur du bloc de traitement 1 n'atteigne l'un des passagers ou usagers. Selon une construction préférée, la chicane 22 est positionnée entre, d'une part, la au moins une source d'émission 5 de lumière et, d'autre part, le au moins un orifice d'entrée 3 et/ou le au moins un orifice de sortie 4 du bloc de traitement 1. A titre d'exemple, au moins une des parois de la chicane 22 est disposée de façon à masquer le au moins un orifice d'entrée 3 et/ou le au moins un orifice de sortie 4 depuis un ou plusieurs points de rayonnement de la source d'émission 5 de lumière. Selon un autre exemple se rapportant à une spécificité de cette variante de construction, le bloc de traitement d'air 1 comprend plusieurs chicanes 22 dont la répartition et le positionnement dans le volume intérieur du bloc de traitement 1 permet d'opérer une combinaison de plusieurs des parois respectives de ces chicanes 22 de façon à masquer le au moins un orifice d'entrée 3 et/ou le au moins un orifice de sortie 4 depuis un ou plusieurs points de rayonnement de la source d'émission 5 de lumière. Selon un autre exemple se rapportant à une autre spécificité de cette variante de construction, au moins une surface de cette chicane est également susceptible d'être adaptée à la réflexion d'au moins une partie du rayonnement de longueur d'onde comprise entre 100 nm et 400 nm, de façon à optimiser la retenue des rayonnements à l'intérieur du bloc de traitement 1.

L'intégration d'une ou plusieurs chicanes 22 à l'intérieur du bloc de traitement 1 apporte également comme avantage d'autoriser un rayonnement UV qui présente une intensité et une puissance élevées dans le volume intérieur 21 du bloc de traitement 1. A titre d'exemple, ce rayonnement est susceptible d'être effectué de façon à fournir une quantité d'énergie de l'ordre d'au moins 3 mJ/cm², idéalement au moins 3,5 mJ/cm². De même, à titre d'exemple, ce rayonnement est susceptible d'être effectué de façon à fournir une intensité d'énergie de l'ordre d'au moins 900 µW/cm², idéalement au moins 950 µW/cm². Ces exemples de rayonnement effectués pendant une durée de l'ordre d'au moins 30 secondes sont ainsi susceptibles de présenter une action virucide sur le flux d'air traité de l'ordre d'au moins 99 %, voire même 99,99 %.

Selon un exemple de construction se rapportant à une autre variante de réalisation de l'invention susceptible d'être combinée avec les différentes variantes de réalisation précédentes, le bloc de traitement 1 comprend au moins une source ou au moins un ensemble de sources d'émission 5 de lumière de longueur d'onde comprise entre 100 nm et 400 nm disposée au niveau d'une section du parcours de circulation d'air à l'intérieur du volume 21 du bloc 1 située à proximité d'un orifice d'entrée 3 respectif. Ce positionnement particulier d'au moins une source d'émission 5 de lumière UV à l'intérieur du bloc de traitement 1 réalise un arrangement dans lequel l'effet germinicide ou virucide du rayonnement est appliqué au flux d'air au plus près de son entrée à l'intérieur du bloc de traitement 1, de façon à profiter de cet effet sur une portion maximale du parcours de circulation d'air, à l'intérieur du bloc de traitement 1. Par ailleurs, le positionnement d'une source d'émission 5 de lumière UV à proximité de chacun des orifices d'entrée 3 du bloc 1 permet d'opérer un traitement germinicide/virucide spécifique, voire adapté, à chacun des flux d'air entrant. Selon un exemple préféré de construction, une source d'émission 5 de lumière est associée à chaque orifice d'entrée 3 du bloc 1 de sorte que le volume intérieur 21 du bloc 1 de traitement soit couvert par le rayonnement de ces sources d'émission 5.

Selon un exemple de construction se rapportant à une autre variante de réalisation de l'invention susceptible d'être combinée avec les différentes variantes de réalisation précédentes, le bloc de traitement 1 est réalisé sous la forme d'une cartouche 2 qui présente un arrangement sensiblement aplati définissant, d'une part, un cadre périphérique 23 et, d'autre part, deux larges surfaces 24 opposées, respectivement entourées par ce cadre périphérique 23 et positionnées de part et d'autre de la cartouche 2, de sorte qu'au moins une de ces deux surfaces 24 comprend au moins un orifice de sortie 4. Selon cet exemple de construction, l'arrangement aplati de la cartouche 2 permet la réalisation d'un bloc de traitement 1 qui présente un encombrement restreint de sorte que celui-ci est aisément positionnable à l'intérieur d'une ventilation existante, notamment au niveau d'une section d'une partie du circuit de déplacement du flux d'air dans cette ventilation. Cet arrangement sensiblement plan de la cartouche 2 permet également le positionnement du bloc de traitement 1 de façon à obstruer une section du flux de circulation d'air dans une ventilation, de sorte que au moins une partie du flux d'air dans cette ventilation est contrainte à un déplacement au travers du bloc de traitement 1. Le positionnement de l'orifice de sortie 4 de l'air de la cartouche 2 au niveau de l'une des deux surfaces 24 permet également d'orienter le flux d'air en sortie selon une direction sensiblement orthogonale au plan moyen du bloc de traitement 1, de sorte que l'air, en sortie du bloc de traitement 1, se trouve réintroduit efficacement dans le circuit de ventilation. Selon un exemple se rapportant à une spécificité de cette variante de construction, l'orifice de sortie 4 est porté par une structure qui forme une aile, par exemple sous la forme d'une excroissance, au niveau du cadre périphérique 23 de la cartouche 2. L'orifice de sortie 4 se trouve alors positionné dans le plan de l'une des surfaces 24 de la cartouche 2, de sorte que le flux soit orienté selon une direction sensiblement orthogonale au plan moyen du bloc de traitement 1. Selon un exemple se rapportant à une variante de construction, l'orifice d'entrée 3 est également positionné sur l'une des surfaces 24 de la cartouche 2, préférentiellement sur une surface différente de celle qui porte l'orifice de sortie 4.

Selon un exemple de construction se rapportant à une autre variante e réalisation de l'invention susceptible d'être combinée avec la variante de réalisation précédemment détaillée, la cartouche 2 comprend au moins un orifice d'entrée 3 positionné au niveau d'au moins une portion de la surface périphérique du cadre 23. La direction du flux d'air qui entre dans la cartouche 2 est ainsi orientée selon un axe sensiblement orthogonal à l'axe de la direction du flux d'air en sortie de la cartouche 2. Cette différence d'orientation entre le flux d'air entrant et le flux d'air sortant permet, dans le volume intérieur 21 de la cartouche 2 lorsque que celui-ci est plus important que les orifices d'entrée 3 et de sortie 4 de la cartouche 2, de générer différentes turbulences dans ce volume intérieur 21, de sorte que les germes et virus en suspension sont susceptibles d'être présents plus longtemps dans ce volume intérieur 21 et de supporter une exposition pendant une durée plus longue aux rayonnements UV de la source d'émission 5 de lumière. De même, cette différence d'orientation permet d'opérer un ralentissement de la circulation du flux d'air à l'intérieur de la cartouche 2, de sorte que les germes et virus en suspension supportent, là encore, une exposition pendant une durée plus longue aux rayonnements UV. Selon un exemple spécifique de construction, l'orifice d'entrée 3 porté par le cadre périphérique 23 comprend au moins un arrangement grillagé ou rainuré configuré pour présenter une construction réalisant une pluralité de chicanes juxtaposées et réparties sur la longueur de l'orifice d'entrée 3. Cet arrangement grillagé ou rainuré sous la forme de chicanes permet ainsi la réalisation d'un flux d'air entrant vers l'intérieur de la cartouche 2 tout en évitant qu'une partie du rayonnement UV apporté par la source d'émission 5 de lumière à l'intérieur de la cartouche 2 ne soit émis vers l'extérieur du bloc de traitement 1. A titre d'exemple de construction, ces chicanes portés par l'orifice d'entrée 3 présentent la forme d'une ou de plusieurs rangés de volets fixes orientés de façon sensiblement parallèle entre eux selon un arrangement similaire à celui de persiennes.

Selon un exemple de construction se rapportant à une autre variante de réalisation de l'invention susceptible d'être combinée avec les différentes variantes de réalisation précédentes, le bloc de traitement 1 comprend également au moins une interface de fixation positionnée sur une partie de la périphérie de la cartouche 2 et/ou au moins une interface de connexion de la source d'émission 5 de lumière à un réseau d'alimentation. Cette interface de fixation permet ainsi l'intégration et le blocage en position du bloc de traitement 1 dans un équipement de ventilation. A titre d'exemple, cette interface de fixation est susceptible d'être réaliser sous la forme d'une interface de vissage ou d'un mécanisme de clipsage permettant un assemblage facilité du bloc de traitement 1 au dispositif de ventilation 6 et notamment au niveau d'un orifice de circulation du flux d'air dans le dispositif 6. L'interface de connexion permet l'alimentation d'au moins une des sources 5 lumineuses du bloc de traitement 1 à partir d'un réseau d'alimentation extérieur au bloc de traitement de sorte que le fonctionnement du bloc de traitement 1 ne nécessite pas la présence d'une source d'énergie embarquée. En profitant d'une alimentation extérieure par cette interface de connexion, la réduction de l'encombrement du bloc de traitement 1 se trouve également en mesure d'être optimisée.

Selon un exemple de construction se rapportant à une alternative de construction à la variante de de réalisation précédemment détaillées, le bloc de traitement 1 comprend au moins une unité embarquée pour l'alimentation des sources 5 lumineuses du bloc de traitement 1. A titre d'exemple, cette unité embarquée est susceptible de prendre la forme d'une ou de plusieurs batteries intégrées au bloc de traitement 1 et spécifiquement dédiées à une source 5 lumineuse ou un groupe de plusieurs sources 5 lumineuses du bloc de traitement 1.

L'invention porte également sur un dispositif de ventilation 6, caractérisé en ce que le dispositif de ventilation 6 intègre au moins un bloc de traitement 1 selon l'invention. Cette intégration du bloc de traitement 1 est opérée de sorte que le bloc de traitement 1 obture une section d'au moins une partie du parcours de circulation de l'air dans le dispositif de ventilation 6. Cette obturation de la section de cette portion du parcours permet ainsi d'assurer une intervention du bloc de traitement 1 sur l'ensemble du flux d'air en déplacement au travers de ladite portion du parcours. Le bloc de traitement 1 réalise un élément structurellement indépendant du reste du dispositif de ventilation 6. Le déplacement du flux d'air est effectué par un ou plusieurs mécanismes de ventilation propre au dispositif de ventilation 6, le bloc de traitement 1 étant dépourvu d'un quelconque mécanisme de ventilation. A titre d'exemple, le dispositif de ventilation 6 est configuré pour générer un débit d'air de l'ordre de 834 m³ par heure, de sorte que pour une application à un habitacle 7 de véhicule de transport en commun, l'intégralité du volume d'air de l'habitacle 7 est en mesure d'être renouvelé en moins de 5 minutes, voire même lors d'un cycle de ventilation d'une durée de l'ordre de 3 à 2 minutes.

Il convient de relever que, pour des raisons de sécurité, l'accès au bloc de traitement 1 dans le dispositif de ventilation 6 est associé à un mécanisme de rupture du fonctionnement du bloc de traitement 1 et notamment de coupure de l'alimentation des différentes sources 5 lumineuses. Ainsi, la manutention du bloc de traitement 1 se réalise sans risque pour un manipulateur. De façon complémentaire, le fonctionnement des différentes sources 5 lumineuses du bloc de traitement 1 est associé à un signal lumineux dans le spectre du visible, de façon à prévenir un éventuel manipulateur que l'alimentation des différentes sources 5 lumineuses n'est pas rompu et qu'une manutention du bloc de traitement 1 est susceptible de présenter un danger.

Selon un exemple de construction se rapportant à une variante de réalisation de l'invention, le dispositif de ventilation 6 étant associé à un habitacle 7 de façon à opérer une ventilation de l'air de l'habitacle 7, au moins un bloc de traitement 1 est positionné sur le parcours de circulation de l'air dans le dispositif de ventilation 6 entre au moins un orifice d'entrée 63 et au moins un orifice de sortie 64, de sorte que l'intégralité de l'air circulant dans le dispositif de ventilation 6 en provenance de l'habitacle 7 traverse au moins un bloc de traitement 1. Selon un exemple de construction, le dispositif de ventilation 6 comprend un orifice d'entrée 63 unique et/ou un orifice de sortie 64 unique du flux d'air au niveau duquel est positionné un bloc de traitement 1 qui permet le traitement de l'ensemble du flux d'air qui transite au travers du dispositif de ventilation 6. Selon un autre exemple de construction, le dispositif de ventilation 6 comprend plusieurs orifices d'entrée 63 et/ou plusieurs orifices de sortie 64 au niveau de chacun desquels, en entrée ou en sortie, un bloc de traitement 1 est positionné. Selon chacun de ces exemples, l'ensemble du flux d'air en déplacement à l'intérieur du dispositif de ventilation 6 est conduit à traverser au moins un bloc 1 de l'invention de façon à y être traité.

Selon un autre exemple de construction se rapportant à une variante de réalisation de l'invention susceptible d'être combiné avec l'une des variantes précédentes, le dispositif de ventilation 6 étant associé à un habitacle 7 de façon à opérer une ventilation de l'air de l'habitacle 7, le dispositif de ventilation comprend également au moins un orifice d'entrée 65 d'air en provenance de l'extérieur de l'habitacle 7 et à destination de l'intérieur de l'habitacle 7. Selon cette variante de construction, le circuit du flux d'air dans le dispositif de ventilation 6 est configuré de sorte que l'air en provenance de l'intérieur de l'habitacle 7 et traité par un bloc de traitement 1, pour être mélangé avec un flux d'air en provenance de l'extérieur du véhicule, avant d'être ventilé vers l'intérieur de l'habitacle 7. Outre le renouvellement de l'air dans l'habitacle 7, cette variante de construction permet également d'opérer une ventilation vers l'intérieur de l'habitacle 7 qui permette de générer une légère surpression à l'intérieur de l'habitacle 7, cette surpression s'équilibrant notamment par un phénomène d'expulsion d'air depuis l'intérieur de l'habitacle 7, d'une part, au niveau des différentes jonctions d'éléments de l'habitacle 7 dépourvus d'étanchéité et, d'autre part, au moment de l'ouverture des portes de l'habitacle 7 notamment à chacun des arrêts du véhicule lorsque celui-ci est destiné à du transport en commun.

Bien entendu, l'invention n'est pas limitée aux modes de réalisation décrits et/ou représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Bloc de traitement d'air (1) réalisé sous la forme d'une cartouche (2) configurée pour être positionnée sur au moins une partie du parcours de circulation d'air dans un équipement de ventilation, ce bloc de traitement (1) comprenant un volume intérieur (21) associé à au moins un orifice d'entrée (3) et au moins un orifice de sortie (4) positionnés aux extrémités d'un parcours de circulation d'air, **caractérisé en ce que** au moins une partie de la surface intérieure du volume (21) est équipée d'au moins une source d'émission (5) de lumière de longueur d'onde comprise entre 100 nm et 400 nm arrangée pour rayonner vers l'intérieur du volume du bloc (1) au niveau d'au moins une section du parcours de circulation d'air à l'intérieur du volume du bloc (1).

2. Bloc de traitement d'air (1) selon la revendication 1, **caractérisé en ce que** au moins une partie de la surface intérieure du volume intérieur (21) est réalisée sous la forme d'une surface adaptée à la réflexion d'au moins une partie du rayonnement de longueur d'onde comprise entre 100 nm et 400 nm, de sorte que ce rayonnement converge au niveau d'au moins une section du parcours de circulation d'air à l'intérieur du volume intérieur (21) du bloc (1).

3. Bloc de traitement d'air (1) selon une des revendications précédentes, **caractérisé en ce que** le parcours de circulation d'air à l'intérieur du volume du bloc (1) comprend au moins une chicane (22) positionnée dans le volume intérieur (21), de sorte que aucun rayon provenant de la au moins une source d'émission (5) de lumière soit en mesure de traverser le au moins un orifice d'entrée (3) et/ou le au moins un orifice de sortie (4) du bloc de traitement (1).

4. Bloc de traitement d'air (1) selon une des revendications précédentes, **caractérisé en ce que** le bloc (1) comprend au moins une source ou au moins un ensemble de sources d'émission (5) de lumière de longueur d'onde comprise entre 100 nm et 400 nm disposée au niveau d'une section du parcours de circulation d'air à l'intérieur du volume (21) du bloc (1) située à proximité d'un orifice d'entrée (3) respectif.

5. Bloc de traitement d'air (1) selon une des revendications précédentes, **caractérisé en ce que** la cartouche (2) présente un arrangement sensiblement aplati définissant, d'une part, un cadre périphérique (23) et, d'autre part, deux larges surfaces (24) opposées, respectivement entourées par ce cadre périphérique (23) et positionnées de part et d'autre de la cartouche (2), de sorte qu'au moins une de ces deux surfaces (24) comprend au moins un orifice de sortie (4).

6. Bloc de traitement d'air (1) selon la revendication 5, **caractérisé en ce que** la cartouche (2) comprend au moins un orifice d'entrée (3) positionné au niveau d'au moins une portion de la surface périphérique du cadre (23).

7. Bloc de traitement d'air (1) selon une des revendications précédentes, **caractérisé en ce que** le bloc de traitement (1) comprend également au moins une interface de fixation positionnée sur une partie de la périphérie de la cartouche (2) et/ou au moins une interface de connexion de la source d'émission (5) de lumière à un réseau d'alimentation.

8. Dispositif de ventilation (6), **caractérisé en ce que** le dispositif de ventilation (6) intègre au moins un bloc de traitement d'air (1) selon une des revendications 1 à 7.

9. Dispositif de ventilation (6) selon la revendication 8, **caractérisé en ce que**, le dispositif de ventilation (6) étant associé à un habitacle (7) de façon à opérer une ventilation de l'air de l'habitacle (7), au moins un bloc de traitement (1) est positionné sur le parcours de circulation de l'air dans le dispositif de ventilation (6) entre au moins un orifice d'entrée (63) et au moins un orifice de sortie (64), de sorte que l'intégralité de l'air circulant dans le dispositif de ventilation (6) en provenance de l'habitacle (7) traverse au moins un bloc de traitement (1).

10. Dispositif de ventilation (6) selon une des revendications 8 ou 9, **caractérisé en ce que**, le dispositif de ventilation (6) étant associé à un habitacle (7) de façon à opérer une ventilation de l'air de l'habitacle (7), le dispositif de ventilation (6) comprend également au moins un orifice d'entrée (65) d'air en provenance de l'extérieur de l'habitacle (7) et à destination de l'intérieur de l'habitacle (7).
